**Europäisches Patentamt**

**European Patent Office**

**Office européen des brevets**

(19)

(11) Veröffentlichungsnummer: **0 164 657**
**B1**

(12) # EUROPÄISCHE PATENTSCHRIFT

(45) Veröffentlichungstag der Patentschrift:
**19.04.89**

(21) Anmeldenummer: **85106658.9**

(22) Anmeldetag: **30.05.85**

(51) Int. Cl.⁴: **C 12 P 19/18,** C 08 B 37/02,
A 61 K 31/715

(54) Wasserlösliches Eisendextran und Verfahren zu seiner Herstellung.

(30) Priorität: **15.06.84 DE 3422249**

(43) Veröffentlichungstag der Anmeldung:
**18.12.85 Patentblatt 85/51**

(45) Bekanntmachung des Hinweises auf die Patenterteilung:
**19.04.89 Patentblatt 89/16**

(84) Benannte Vertragsstaaten:
**AT BE CH DE FR GB LI NL SE**

(56) Entgegenhaltungen:
BE-A-674 385
BE-A-723 549

**CHEMICAL ABSTRACTS, Band 94, Nr. 10, 9. März 1981, Seite 360, Zusammenfassung Nr. 71270q, Columbus, Ohio, US; UNITED STATES FOOD AND DRUG ADMINISTRATION: "Oral dosage form new animal drugs not subject to certification; iron dextran oral suspension", & FED. REGIST. 14-11-1980, 45(222), 75199**

**Die Akte enthält technische Angaben, die nach dem Eingang der Anmeldung eingereicht wurden und die nicht in dieser Patentschrift enthalten sind.**

(73) Patentinhaber: **PFEIFER & LANGEN, Linnicher Strasse 48, D-5000 Köln 41 (DE)**

(72) Erfinder: **Schwengers, Dieter, Dr., Jussenhovener Strasse 37, D-4047 Dormagen 1 (DE)**

(74) Vertreter: **Eggert, Hans- Gunther, Dr., Räderscheidtstrasse 1, D-5000 Köln 41 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

## Beschreibung

Eisendextranpräparate zur Behandlung von Eisenmangel-Anämien bei Mensch und Tier sind bekannt. Sie werden vor allem in der Tiermedizin intravenös und intramuskulär injiziert. Die Präparate werden als Komplex des Dextrans durch Umsetzung mit colloidalem Eisen-III-hydroxid hergestellt.

Besonders an Präparate zur intravenösen Verabreichung werden hohe Anforderungen in Bezug auf die Stabilität der Lösung und die Bioverfügbarkeit des Eisens gestellt. Bei Eisendextranen mit hohem Eisengehalt, d. h. mit 20 % und mehr Eisen pro Gramm Trockensubstanz sind diese Forderungen nicht erfüllbar, wenn für die Herstellung Dextrane mit zu hohem mittleren Molekulargewicht verwendet werden. Infolge intermolekularer Komplexbildung über die Eisenatome zwischen verschiedenen Dextranmolekülen kommt es dann zu Gel- und Präzipitatbildung. Daher werden für die synthese von Eisendextran üblicherweise Dextrane niedriger Molekulargewichte von 4 000 - 6 000 verwendet. Dextrane dieses Molekulargewichtsbereichs werden durch Säurehydrolyse von hochmolekularem nativem Dextran erhalten und fallen z. B. bei der Herstellung klinischer Dextrane, die ein mittleres Molekulargewicht von 40 000 - 75 000 haben, als Nebenprodukt an.

Die Molekulargewichtsverteilung dieser "Abfalldextrane" ist jedoch sehr breit und reicht üblicherweise von Glucose bis zu Molekulargewichten von etwa 50 000. Es ist daher eine allgemeine Praxis, das durch Umsetzung von Dextran und Fe(III)Salzen unter alkalischen Bedingungen erhaltene Roh-Eisendextran durch aufwendige Fällungsfraktionierungen mit Lösungsmitteln oder durch Membrantrennverfahren von unerwünschten höhermolekularen Anteilen zu befreien.

Enthalten die Abfallfraktionen der Herstellung von klinischem Dextran zuviel Glucose und iso-Malto-Oligosaccharide bis zu einem Molgewicht von etwa 1.000, müssen diese Saccharide vor der Umsetzung zum Eisendextran ebenfalls entfernt werden, da sie sich bei den Umsetzungsbedingungen unter Bildung toxischer Produkte weitgehend zersetzen.

Aus der GB-PS 1 200 902 ist ein wasserlösliches Dextran mit einem Molekulargewicht von 3000 bekannt, jedoch handelt es sich dort um ein mit Brom oxidiertes Dextran.

In einem aus der belgischen Patentschrift 723 549 bekannten Verfahren werden ebenfalls nur oxidierte Dextrane, die durch Oxidation des Dextrans mit Brom erhalten wurden, eingesetzt.

Aus der belgischen Patentschrift 674 385 ist bekannt, daß Dextrane mit einer Intrinsic-Viskosität von 0,75 bis 0,025, entsprechend Molekulargewichten von etwa 700.000 bis etwa 600 verwandt werden können. Der Einsatz in bezug auf das Molekulargewicht beliebiger Dextrane wird dadurch erreicht, daß nach einer Teilneutralisation einer Eisensalzlösung auf einen pH-Wert von 1,1 bis 2,3 das Dextran hinzugegeben wird und die resultierende noch saure Eisen-Dextran-Komplexlösung im Autoklaven erhitzt wird, bis durch Depolymerisation des Dextrans die gewünschte Viskosität der Eisen-Dextranlösung erreicht ist. Es ist somit keine Aussage über das Molekulargewicht des Dextrans im Eisen-Dextran-Komplex möglich. Das Verfahren hat außerdem den Nachteil, daß vor dem depolymerisierenden Kochschritt toxische Eisen-Verbindungen durch aufwendige Fällungsfraktionierungen mit Lösungsmitteln entfernt werden müssen.

Aus den genannten Gründen wäre es wünschenswert, ein wasserlösliches Eisendextran mit hohem Eisengehalt herzustellen, was wiederum bedeutet, daß man über Dextrane eines mittleren Molekulargewichts von 2 000 bis 4 000 mit enger Molekulärgewichtsverteilung verfügen muß.

Diese Aufgabe wird durch das erfindungsgemäße Verfahren gelöst welches sich dadurch auszeichnet, daß man einer wässrigen Lösung die mehr als 200 mMol D-Glucose pro 1 000 U α (1→6)-D-Glucosyltransferase enthält bei -8°C bis 37°C und einem pH-Wert von 4,5 bis 8 einen wässrige Saccharoselösung in einer solchen Menge zugibt, daß das molare Verhältnis von Saccharose zu Glucose 2,0 bis 5,0 beträgt, nach Verbrauch der Saccharose, die Glucose, freigesetzte Fructose und unerwünschte Oligosaccharide abtrennt, das so gereinigte Dextran mit einer mittleren Molmasse von 2 000 bis 4 000 mit frisch gefälltem Eisen-III-Hydroxid umsetzt und ggf. weiter reinigt.

Auf diese Weise gelingt es, ein wasserlösliches Eisendextran mit einem Eisengehalt von 27 bis 33 Gewichtsprozent und einer mittleren Molmasse des Dextrananteils von 2 000 bis 4 000 herzustellen.

Das Reaktionsgemisch wird vorzugsweise bei 17°C bis 27°C und in einem pH-Wert-Bereich von 5 bis 6,5 gehalten. Beide Paramater haben einen Einfluß auf die Struktur der entstehenden Produkte.

Als α (1 → 6)-D-Glucosyltransferase nach der Klassifizierung der "Enzyme Commission" bezeichnet man Enzyme, die die D-Glucopyranosylgruppe der Saccharose auf geeignete Akzeptoren übertragen. Ein solches extracelluläres Enzym ist Dextransucrase (E.C. 2.4.1.5) das von gewissen Bakterienarten der Lactobacilli-Familie, z. B. Leuconostoc mesenteroides, insbesondere dem Stamm B-512, (hinterlegt in der American Type Culture Collection unter der Stammnummer ATCC 10830 bzw. 10830a) Leuconostoc dextranicum, Streptococcus und Lactobacillus gebildet wird. Zur Herstellung von nativem Dextran dient in erster Linie Saccharose als Akzeptor und wirkt als Ketteninitiator für eine Kettenpolymerisation, bei der durch fortlaufende Übertragung von D-Glucopyranosyl-Gruppen aus der Saccharose auf die wachsende Kette das Polysaccharid Dextran mit Molmassen von mehreren Millionen gebildet wird, während gleichzeitig für jedes Molekül umgesetzter Saccharose ein Molekül Fructose frei wird.

Wenn man bei dieser Reaktion andere Mono-, Di- oder Tri-saccharide als Akzeptor einsetzt, werden auf Kosten des Dextrans in geringem Umfang Oligosaccharide gebildet. Bei Einsatz von Glucose als Akzeptor entstehen etwa 78 % natives Dextran und als Nebenprodukt etwa 13 % Di- und Oligosaccharide bis IM-12. (Robyt und Eklund, Carbohydrate Research 121 (1983) 279 - 286). Typischerweise werden die Oligosaccharide mit steigendem Polymerisationsgrad in abnehmender Menge gebildet.

Unter den erfindungsgemäßen Reaktionsbedingungen gelingt es, die Übertragung von Glucosyl-Gruppen aus der Saccharose auf Glucose so zu lenken, daß kein natives Dextran mehr entsteht, sondern in hoher Ausbeute die iso-Malto-Oligosaccharide mit 15 bis 25 Anhydroglucose-Einheiten aufgebaut werden. Überraschend dabei ist, daß die iso-Malto-Oligosaccharide mit steigendem Polymerisationsgrad nicht mehr in abnehmender Menge gebildet werden, sondern daß in Abhängigkeit von der umgesetzten Saccharosemenge ein bestimmter Polymerisationsgrad bevorzugt gebildet wird.

Bei dem erfindungsgemäßen Verfahren empfiehlt es sich zur Erzielung hoher Ausbeuten der gewünschten iso-Malto-Oligosaccharide die wässrige Lösung von Saccharose mit einer solchen Geschwindigkeit kontinuierlich zuzugeben, daß die vorgelegte Enzymmenge die zulaufende Saccharosemenge sofort umsetzen kann und so eine Anreicherung von Saccharose im Reaktionsgemisch, die zur unkontrollierten Bildung von hochmolekularem Dextran führen kann, vermieden wird. In jedem Fall sollte der Anteil der Saccharose an der Kohlehydrat-Trockensubstanz des Reaktionsgemisches im Gleichgewichtszustand der kontinuierlichen Umsetzung 25 % nicht überschreiten.

Anstelle der gereinigten Dextran-Sucrase kann auch das Gemisch aus dem Enzym und dem es produzierenden Bakterien eingesetzt werden.

Die Aufbaureaktion läßt sich wie folgt darstellen:

FIG00/10
iso-Malto-(n + 1)-Saccharide + n Fructose

Hierin bedeutet n die Anzahl der Mole der eingesetzten Saccharose, deren D-Glucopyranosyl-Gruppen zum Aufbau des niedermolekularen Dextrans dienen, während eine entsprechende Molzahl Fructose freigesetzt wird.

Diese Reaktion läßt sich erfindungsgemäß so steuern, daß iso-Malto- Oligo- oder Polysaccharide des jeweils gewünschten Molekulargewichts erhalten werden. Unter den genannten Bedingungen der Temperatur und der Wasserstoffionenkonzentration hängt das bei dieser Aufbausynthese erreichte Molekulargewicht von der auf eine bestimmte Enzymaktivität, in der vorgelegten Lösung bezogenenen molaren Menge des Akzeptors und dem Molverhältnis der insgesamt zugeführten Saccharose zum Akzeptor ab.

Die Enzymaktivitätseinheit U (= Unit) ist die Menge der α(1 → 6)-D-Glucosyltransferase, die 1 μMol Saccharose pro Minute bei pH 5,2 und 25°C umsetzt. Wenn mehr Saccharose zugeführt wird, als die vorgelegte Enzym-Aktivität umsetzen kann, ist die Steuerung der Molekülgröße nicht mehr möglich.

Legt man als Enzymaktivität 1 000 U zugrunde, so wird bei eine Saccharosezusatz von insgesamt 1 000 mMol und mehr als 200 mMol, z. B. 400 bis 600 mMol Glucose pro 1 000 Units das gewünschte Oligo-Saccharidgemisch mit einem mittleren Molekulargewicht von etwa 2 000 bis 4 000 erhalten.

Es ist also möglich, in wenigen Vorversuchen mit wechselnden molaren Mengen an Glucose innerhalb der aufgezeigten Bereiche bei vorgegebener Aktivität der α(1 → 6)-D-Glucosyltransferase (z. B. 1 000 U) und einer konstanten Menge Saccharose (z. B. 1 000 mMol), die in dem Maße zugegeben wird, daß sie direkt von dem Enzym umgesetzt wird, die Anlagerung der D-Glucopyranosyl-Gruppen der Saccharose an die Glucose als Akzeptor so zu steuern, daß in hoher Ausbeute Fraktionen der jeweils gewünschten iso-Malto-Oligo- bzw. Polysaccharide mit enger Molekulargewichtsverteilung synthetisiert werden können.

Man kann die gesamte erforderliche Glucosemenge vorlegen oder unter Wahrung der übrigen Reaktionsbedingungen, insbesondere der Konzentrationsverhältnisse auch die Glucose kontinuierlich in dem Maße ersetzen, wie sie als Akzeptor verbraucht wird. Es ist also möglich, die Synthesereaktion voll kontinuierlich durchzuführen.

Es ist ein unerwarteter Vorteil der erfindungsgemäßen Verfahrensweise, daß der Kohlehydrat-Trockensubstanz-Gehalt des Reaktionsgemisches sehr hoch sein kann, indem er 30 bis 50 %, insbesondere 40 bis 50 % beträgt.

Obgleich bei der erfindungsgemäßen enzymatischen Aufbausynthese steril gearbeitet wird, wie das z. B. für die Synthese von nativem Dextran üblich ist, können dem Reaktionsgemisch zur Vermeidung von unerwünschtem Hefewachstum Antimitotika (Mytosehemmer), wie schweflige Säure in Mengen bis zu 1 000 mg/kg, insbesondere 400 bis 600/mg/kg zugesetzt werden.

Die Gewinnung der Dextrane mit niedrigem mittlerem Molekulargewicht von 2000 bis 4000 aus dem Reaktionsgemisch durch Abtrennung der nicht umgesetzten Glucose, der freigesetzten Fructose, und der iso-Malto-Oligo-saccharide mit weniger als 6 Anhydroglucose-Einheiten kann nach an sich bekannten Methoden, z. B. durch Fällungsfraktionierung mit Ethanol erfolgen.

Als sehr geeignet hat sich die Abtrennung der Nebenprodukte durch Chromatographie über eine mit einem stark sauren Kationenaustauscher gefüllte Säule erwiesen.

Zur Herstellung des Eisen-Dextrans wird dann eine wässrige Aufschlämmung des iso-Malto-Oligo-saccharidgemisches mit frisch gefälltem, gewaschenem Eisen-III-Hydroxid auf Temperaturen bis zu 100° C erhitzt bis das Eisen-III-Hydroxid in Lösung gegangen ist. Es empfiehlt sich, diese Umsetzung in Gegenwart von Citronensäure oder Alkalicitrat durchzuführen, wie das aus der DE-PS 1 768 912 an sich bekannt ist.

Zur Entfernung von An- oder Kationen, die noch in der nach der Umsetzung erhaltenen Lösung des Eisendextrans vorliegen, wird dieses der Einwirkung von Kationen- und Anionenaustauschern unterworfen.

**Beispiel**

Durch Zutropfen einer Lösung von 28 g Soda, gelöst in 600 ml Wasser, zu 250 ml einer wässrigen Lösung von 43 g Eisen(III)chlorid-Hexahydrat wurde Eisen(III)hydroxid ausgefällt. Das Eisen(III)hydroxid wurde abfiltriert und mit destilliertem Wasser gewaschen. Das Eisen (III) hydroxid wurde in einen 1 l Rührkolben überführt und mit 15 g des durch Chromatographie erhaltenen Oligosaccharidgemisches, einer mittleren Molmasse von 3 000 g/Mol gemäß Beispiel 2 sowie 0,5 g Citronensäure gemischt. Nach Zugabe von 9 ml einer 20 % NaOH-Lösung wurde das Gemisch bei 95°C - 100°C solange gerührt, bis das Eisen(III)hydroxid vollständig in Lösung gegangen war.

Die tiefdunkelrote Lösung des entstandenen Eisendextrans wurde auf Zimmertemperatur abgekühlt und mittels Ionenaustauschern vollentsalzt. Nach einer Sterilfiltration wurde die Lösung durch Eindampfen auf einen Gehalt von 10 % Eisen/ml konzentriert und steril in Ampullen abgefüllt. Der Eisengehalt auf Trockensubstanz bezogen, betrug 29,5 %.

Die erhaltene Eisendextranlösung wurde nach dem British Veterinary Codex von 1965 in Bezug auf Toxizität geprüft.

Für den Versuch wurden NMRI-Mäuse (Zucht: Winkelmann, Paderborn) mit einem Durchschnittsgewicht von 20 g verwendet.

Die Testsubstanz rief in der geprüften Dosierung von 0,25 ml/Tier sofort nach Beendigung der Injektion leichte Ataxien hervor, die aber innerhalb von 1 h p. a. wieder völlig abgeklungen waren. Über den restlichen Nachbeobachtungszeitraum wurden keine Symtome mehr festgestellt. Es traten keine Mortalitäten auf.

**Beispiel 2**

In 16 l einer wässrigen Lösung des Enzyms Dextransucrase, die eine Aktivität von 5 400 U/l hatte, wurden 7,3 kg kristalline Glucose bei 25°C gelöst. Der pH-Wert der Lösung betrug 5,4. Zu dieser Lösung wurden kontinuierlich 2,6 kg/h einer 40 % Saccharoselösung mit einem pH-Wert von 5,4 gepumpt. Nach 48 h wurde die Zugabe der Saccharose beendet und das Enzym nach weiteren 2 Stunden durch Erwärmen des Reaktionsgemisches auf 70°C inaktiviert.

Aus einer Probe des Reaktionsgemisches wurden durch Gelchromatographie der Mono- und Disaccharidanteil abgetrennt und das Zahlenmittel $M_n$ des Molekulargewichts der Oligosaccharidfraktion nach der Somogyi-Phosphat-Methode (Method in Carbohydrate Chemistry, Vol.I, (1962), S. 384 - 386) bestimmt. Es betrug $M_n$ = 2 540, was einem mittleren Polymerisationsgrad von 15,7 Anhydroglucoseeinheiten entspricht. Der Fruktoseanteil an der Kohlehydrattrockensubstanz betrug 45,0 %, der Glucoseanteil 3,6 %.

45 l dieser Saccharidlösung wurden auf eine chromatographische Trennanlage gegeben, die 400 l stark saures, mit Natriumionen beladenes Kationenaustauscherharz enthielt und die einzelnen Saccharide wurden durch Zuführen von 43 l/h destilliertem Wasser aus der Säule eluiert.

Nach einem Vorlauf von 60 l wurde innerhalb der nächsten 22 l aus der Trennsäule ein iso-Malto-Oligosaccharidgemisch eluiert, das eine mittlere Molmasse von 3 000 g/Mol aufwies.

**Versuchsbericht**

Ein Ferkelwurf wurde in zwei Kontrollgruppen zu je vier Tieren eingeteilt. Die eine Gruppe wurde am dritten Lebenstag mit je 2 ml Eisendextran gemäß der GB-PS 1 200 902, die andere Gruppe mit je 2 ml Eisendextran nach der Erfindung gespritzt. Die auftretende Verfärbung des Gewebes an der Injektionsstelle war nach einem Tag bei allen Tieren, denen das erfindungsgemäße Eisendextran gespritzt worden war, verschwunden, während sie bei den mit handelsüblichem Eisendextran gespritzten Ferkeln noch deutlich sichtbar war. Bei diesen Ferkeln war die Verfärbung des Gewebes erst am zweiten Tag nach der Injektion verschwunden.

Dieses Ergebnis belegt, daß das Eisen aus dem erfindungsgemäßen Eisendextran vom blutbildenden Gewebe rascher verwertet wird als dasjenige aus handelsüblichem Eisendextran.

**Patentansprüche**

1. Wasserlösliches Eisendextran mit einem Eisengehalt von 27 bis 33 Gewichtsprozent und einer mittleren Molmasse des Dextrananteils von 2 000 bis 4 000, erhältlich durch Zugabe einer wässrigen Saccharoselösung bei - 8° C bis 37° C und einem pH-Wert von 4,5 bis 8 zu einer wässrigen Lösung von D-Glucose, die pro 1 000 U $\alpha(1 \to 6)$-D-Glucocyltransferase mehr als 200 mMol Glucose enthält, wobei ein Molverhältnis von Saccharose zu Glucose von 2,0 bis 5,0 eingehalten wird, Abtrennung von Glucose, freigesetzter Fructose und unerwünschter Oligosaccharide nach Verbrauch der Saccharose und Umsetzung des iso-Malto-Oligo-saccharid-Gemisches mit frisch gefälltem Eisen-III-Hydroxid.

2. Verfahren zur Herstellung von wasserlöslichem Eisendextran mit einem Eisengehalt von 27 bis 33 Gewichtsprozent und einer mittleren Molmasse des Dextrananteils von 2 000 bis 4 000, dadurch gekennzeichnet, daß man einer wässrigen Lösung, die mehr als 200 mMol D-Glucose pro 1 000 U $\alpha (1 \to 6)$-D-Glucosyltransferase enthält, bei -8° C bis 37° C und einem pH-Wert von 4,5 bis 8 eine wässrige Saccharoselösung in einer solchen Menge zugibt, daß das Molverhältnis von Saccharose zu Glucose 2,0 bis 5,0 beträgt, nach Verbrauch der Saccharose Glucose, freigesetzte Fructose und unerwünschte Oligosacchardide abtrennt, das so gereinigte Dextran mit frisch gefälltem Eisen-III-Hydroxid umsetzt und ggf. weiter reinigt.

3. Verfahren nach Anspruch 2, dadurch gekennzeichnet, daß die wässrige Glucoselösung 400 bis 600 mMol Glucose pro 1 000 U Enzym enthält.

4. Verfahren nach einem der Ansprüche 2 oder 3, dadurch gekennzeichnet, daß die Reaktion bei 17° C bis 27° C durchgeführt wird.

5. Verfahren nach einem der Ansprüche 2 bis 4, dadurch gekennzeichnet, daß der pH-Wert des Reaktionsgemisches 5 bis 6,5 beträgt.

6. Verfahren nach einem der Ansprüche 2 bis 5, dadurch gekennzeichnet, daß man die Saccharoselösung kontinuierlich zugibt.

7. Verfahren nach einem der Ansprüche 2 bis 6, dadurch gekennzeichnet, daß die Saccharoselösung mit einer solchen Geschwindigkeit zugegeben wird, daß die Saccharose direkt von der $\alpha(1 \to 6)$-D-Glucosyltransferase umgesetzt wird.

8. Verfahren nach einem der Ansprüche 2 bis 7, dadurch gekennzeichnet, daß der Kohlehydrattrockensubstanzgehalt des Reaktionsgemisches 30 bis 50 %, insbesondere 40 bis 50 %, beträgt.

9. Verfahren nach einem der Ansprüche 2 bis 8, dadurch gekennzeichnet, daß man als $\alpha (1 \to 6)$-D-Glucosyltransferase die vom Bakterium Leuconostoc mesenteroides, insbesondere dem stamm B-512, ausgeschiedene Dextransucrase verwendet.

10. Verfahren nach einem der Ansprüche 2 bis 8, dadurch gekennzeichnet, daß man als $\alpha (1 \to 6)$-D-Glucosyltransferase die vom Bakterium Leuconostoc dextranicum ausgeschiedene Dextransucrase verwendet.

11. Verfahren nach einem der Ansprüche 2 bis 10, dadurch gekennzeichnet, daß man die D-Glucose kontinuierlich in dem Maße ersetzt, wie sie als Akzeptor verbraucht wird.

12. Verfahren nach einem der Ansprüche 2 bis 11, dadurch gekennzeichnet, daß man dem Reaktionsgemisch zur Vermeidung von unerwünschtem Hefewachstum Antimitotika (Mytosehemmer) zusetzt.

13. Verfahren nach Anspruch 12, dadurch gekennzeichnet, daß man schwefelige Säure in Mengen bis zu 1 000 mg/kg, vorzugsweise 400 bis 600 mg/kg, zusetzt.

14. Verfahren nach einem der Ansprüche 2 bis 13, dadurch gekennzeichnet, daß man aus dem Gemisch der Mono-, Di-, und Oligosaccharide die Dextrane einer mittleren Molmasse von 2 000 bis 4 000 durch Fällungsfraktionierung oder Chromatographie abtrennt.

15. Verfahren nach einem der Ansprüche 2 bis 14, dadurch gekennzeichnet, daß man eine wässrige Aufschlämmung aus dem iso-Malto-Oligosaccharid-Gemisch und dem gefällten Eisen-III-Hydroxid auf Temperaturen bis zu 100° C erhitzt, bis das Eisen-III-Hydroxid in Lösung gegangen ist.

16. Verfahren nach Anspruch 15, dadurch gekennzeichnet, daß man die Umsetzung in Gegenwart von Citronensäure oder Alkalicitrat durchführt.

17. Verfahren nach Anspruch 15 oder 16, dadurch gekennzeichnet, daß man die nach der Umsetzung erhaltene Lösung des Eisendextrans der Einwirkung von Kation- und Anionaustauschern unterwirft.

18. Verwendung einer gereinigten sterilen wässrigen Eisendextranlösung nach Anspruch 1 zur Behandlung von Eisenmangelzuständen.

## Claims

1. A water soluble iron dextran having an iron content of from 27 to 33 percent by weight and an average molar mass of the dextran component of from 2 000 to 4 000, said iron dextran being obtainable by adding an aqueous sucrose solution, at -8° C to 37° C and a pH value of from 4.5 to 8, to an aqueous solution of D-glucose containing per 1 000 U $\alpha (1 \to 6)$-D-glycosyl transferase more than 200 mmol glucose, a mole ratio of sucrose to glucose of from 2.0 to 5.0 being observed; separating the glucose, liberated fructose and undesired oligosaccharides after consumption of the sucrose; and reacting the iso-malto-oligosaccharide mixture with freshly precipitated iron(III)hydroxide.

2. A process for the manufacture of water soluble iron dextran having an iron content of from 27 to 33 percent by weight and an average molar mass of the dextran component of from 2 000 to 4 000, characterized by adding to an aqueous solution containing more than 200 mmol D-glucose per 1 000 U α (1 → 6)-D-glucosyl transferase, at from -8°C to 37°C and a pH value of from 4.5 to 8, an aqueous sucrose solution in such an amount that the mole ratio of sucrose to glucose is from 2.0 to 5.0; separating, after the consumption of the sucrose, liberated fructose and undesirable oligosaccharides; reacting the so-purified dextran with freshly precipitated iron(III)hydroxide and, if desired, further purifying the same.

3. A process according to claim 2, characterized in that the aqueous glucose solution contains from 400 to 600 mmol glucose per 1 000 U enzyme.

4. A process according to any of claims 2 or 3, characterized in that the reaction is conducted at from 17°C to 27°C.

5. A process according to any of claims 2 to 4, characterized in that the pH value of the reaction mixture is from 5 to 6.5.

6. A process according to any of claims 2 to 5, characterized by adding the sucrose solution continuously.

7. A process according to any of claims 2 to 6, characterized by adding the sucrose solution at such a rate that the sucrose is directly converted by the α (1 → 6)-D-glucosyl transferase.

8. A process according to any of claims 2 to 7, characterized in that the dry weight of the carbohydrate content of the reaction mixture is from 30 to 50 percent, preferably from 40 to 50 percent.

9. A process according to any of claims 2 to 8, characterized by using as α (1 → 6)-D-glucosyl transferase the dextran sucrase produced by the bacterium Leuconostoc mesenteroides in particular the strain B-512.

10. A process according to any of claims 2 to 8, characterized by using as α (1 → 6)-D-glucosyl transferase the dextran sucrase produced by the bacterium Leuconostoc dextranicum.

11. A process according to any of claims 2 to 10, characterized by continuously replacing the D-glucose, to the same extent as it is consumed as acceptor.

12. A process according to any of claims 2 to 11, characterized by adding to the reaction mixture antimitotics (mytosis inhibitors) to avoid undesired yeast growth.

13. A process according to claim 12, characterized by adding sulfurous acid in amounts of up to 1 000 mg/kg, preferably from 400 to 600 mg/kg.

14. A process according to any of claims 2 to 13, characterized by separating from the mixture of the mono-, Di-, and oligosaccharides the dextrans having an average molar mass of from 2 000 to 4 000 by precipitation-fractionation or chromatography.

15. A process according to any of claims 2 to 14, characterized by heating an aqueous suspension of the iso-malto-oligosaccharide mixture and the precipitated iron(III)hydroxide to temperatures of up to 100°C until the iron(III)hydroxide is dissolved.

16. A process according to claim 15, characterized by conducting the reaction in the presence of citric acid or alkali citrate.

17. A process according to claim 15 or 16, characterized by subjecting the solution of the iron dextran obtained after the reaction to the action of cation and anion exchangers.

18. The use of a purified sterile aqueous iron dextran solution according to claim 1 for the treatment of iron deficiency.


**Revendications**

1. Complexe ferrique de dextrane hydrosoluble ayant une teneur en fer de 27 à 33 % en poids et une masse moléculaire moyenne de la partie dextrane de 2 000 à 4 000, pouvant être obtenu par addition d'une solution aqueuse de saccharose, à une température de -8°C à 37°C et à une valeur de pH de 4,5 à 8, à une solution aqueuse de D-glucose qui contient, pour 1 000 unités d'α-(1 → 6)-D-glucosyl-transférase, plus de 200 mmoles de glucose, en maintenant un rapport molaire du saccharose au glucose de 2,0 à 5,0, séparation du glucose, du fructose libéré et des oligosaccharides non désirés après consommation du saccharose et réaction du mélange d'iso-malto-oligo-saccharides avec de l'hydroxyde de fer-III fraîchement précipité.

2. Procédé de préparation de complexe ferrique de dextrane hydrosoluble ayant une teneur en fer de 27 à 33 % en poids et une masse moléculaire moyenne de la portion dextrane de 2 000 à 4 000, caractérisé en ce qu'on ajoute une solution aqueuse de saccharose à une solution aqueuse qui contient plus de 200 mmoles de D-glucose pour 1 000 unités d'α (1 → 6)-D-glucosyl-transférase entre -8°C et 37°C et à une valeur de pH de 4,5 à 8, en une quantité choisie de manière que le rapport molaire du saccharose au glucose ait une valeur de 2,0 à 5,0, on sépare le glucose, le fructose libéré et les oligo-saccharides non désirés après consommation du saccharose, on fait réagir le dextrane ainsi purifié avec de l'hydroxyde de fer-III fraîchement précipité et on poursuit éventuellement sa purification.

3. Procédé suivant la revendication 2, caractérisé en ce que la solution aqueuse de glucose contient 400 à 600 mmoles de glucose pour 1000 unités d'enzyme.

4. Procédé suivant l'une des revendications 2 ou 3, caractérisé en ce qu'on conduit la réaction à une température de 17°C à 27°C.

5. Procédé suivant l'une des revendications 2 à 4, caractérisé en ce que la valeur de pH du mélange réactionnel s'élève à 5 - 6,5.

6. Procédé suivant l'une des revendications 2 à 5, caractérisé en ce qu'on ajoute continuellement la solution de saccharose.

7. Procédé suivant l'une des revendications 2 à 6, caractérisé en ce qu'on ajoute la solution de saccharose à une vitesse telle que le saccharose soit directement transformé par l'α (1 → 6)-D-

glucosyl-transférase.

8. Procédé suivant l'une des revendications 2 à 7, caractérisé en ce que la teneur en hydrates de carbone sur base sèche du mélange réactionnel a une valeur de 30 à 50 %, notamment de 40 à 50 %.

9. Procédé suivant l'une des revendications 2 à 8, caractérisé en ce qu'on utilise, comme α (1 → 6)-D-glucosyl-transférase, la dextrane-sucrase qui est extraite de la bactérie Leuconostoc mesenteroides, notamment de la souche B-512.

10. Procédé suivant l'une des revendications 2 à 8, caractérisé en ce qu'on utilise, comme α (1 → 6)-D-glucosyl-transférase, la dextrane-sucrase qui est extraite de la bactérie Leuconostoc dextranicum.

11. Procédé suivant l'une des revendications 2 à 10, caractérisé en ce qu'on remplace continuellement le D-glucose au fur et à mesure qu'il est consommé comme accepteur.

12. Procédé suivant l'une des revendications 2 à 11, caractérisé en ce qu'on ajoute des agents antimitotiques (inhibiteurs de mitose) au mélange réactionnel pour éviter la croissance non désirée de levures.

13. Procédé suivant la revendication 12, caractérisé en ce qu'on ajoute de l'acide sulfureux en quantités allant jusqu'à 1 000 mg/kg, de préférence comprises entre 400 et 600 mg/kg.

14. Procédé suivant l'une des revendications 2 à 13, caractérisé en ce que les dextranes de masse moléculaire moyenne allant de 2 000 à 4 000 sont séparés du mélange des mono-, di- et oligo-saccharides par fractionnement par précipitation, ou par chromatographie.

15. Procédé suivant l'une des revendications 2 à 14, caractérisé en ce qu'on chauffe une suspension aqueuse formée du mélange d'iso-malto-oligosaccharides et de l'hydroxyde de fer-III précipité à des températures allant jusqu'à 100° C, jusqu'à ce que l'hydroxyde de fer-III soit passé en solution.

16. Procédé suivant la revendication 15, caractérisé en ce qu'on conduit la réaction en présence d'acide citrique ou d'un citrate alcalin.

17. Procédé suivant la revendication 15 ou 16, caractérisé en ce qu'on soumet la solution du complexe ferrique de dextrane obtenue après la réaction à l'action d'échangeurs de cations et d'anions.

18. Utilisation d'une solution aqueuse stérile purifiée de complexe ferrique de dextrane suivant la revendication 1 pour le traitement d'états ferriprives.